(19) Europäisches Patentamt / European Patent Office / Office européen des brevets

(11) EP 1 307 501 B1

(12) FASCICULE DE BREVET EUROPEEN

(45) Date de publication et mention de la délivrance du brevet:
**24.10.2012 Bulletin 2012/43**

(21) Numéro de dépôt: **01949591.0**

(22) Date de dépôt: **29.06.2001**

(51) Int Cl.:
***C08G 81/02*** (2006.01) ***C08F 8/32*** (2006.01)
***A61K 8/90*** (2006.01) ***A61K 8/91*** (2006.01)
***A61Q 19/00*** (2006.01)

(86) Numéro de dépôt international:
**PCT/FR2001/002094**

(87) Numéro de publication internationale:
**WO 2002/009064 (31.01.2002 Gazette 2002/05)**

# (54) POLYMERE COMPRENANT DES UNITES HYDROSOLUBLES ET DES UNITES A LCST, ET COMPOSITION AQUEUSE LE COMPRENANT

POLYMERE MIT WASSERLÖSLICHEN EINHEITEN UND LCST EINHEITEN, UND IHRE WÄSSRIGEN ZUSAMMENSETZUNGEN

POLYMER COMPRISING WATER SOLUBLE UNITS AND LCST UNITS, AND AQUEOUS COMPOSITION COMPRISING SAME

(84) Etats contractants désignés:
**AT BE CH CY DE DK ES FI FR GB GR IE IT LI LU MC NL PT SE TR**

(30) Priorité: **21.07.2000 FR 0009614**

(43) Date de publication de la demande:
**07.05.2003 Bulletin 2003/19**

(73) Titulaire: **L'Oréal**
**75008 Paris (FR)**

(72) Inventeur: **L'ALLORET, Florence**
**F-75013 Paris (FR)**

(74) Mandataire: **Dodin, Catherine**
**L'Oréal**
**D.I.P.I.**
**25-29 Quai Aulagnier**
**92600 Asnières (FR)**

(56) Documents cités:
**EP-A- 0 722 994 EP-A- 0 858 795**
**WO-A-97/35814 WO-A-98/31643**

- **HOURDET D ET AL: "Synthesis of thermoassociative copolymers" , POLYMER, ELSEVIER SCIENCE PUBLISHERS B.V,GB, VOL. 38, NR. 10, PAGE(S) 2535-2547 XP004059755 ISSN: 0032-3861 cité dans la demande le document en entier**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a trait à une nouvelle famille de polymères et leurs sels, susceptibles d'être employés dans des compositions cosmétiques ou pharmaceutiques, notamment pour en modifier les propriétés rhéologiques.

Les agents épaississants usuellement employés dans les domaines cosmétiques ou pharmaceutiques, pour contrôler la rhéologie des compositions notamment aqueuses, voient généralement leur viscosité diminuer lorsque la température du milieu augmente. Ce comportement peut toutefois présenter certains inconvénients, tels que la modification de la rhéologie de la composition en fonction des changements de température (compositions qui se fluidifient en été et sont plus visqueuses en hiver).

On connaît dans l'état de la technique des polymères particuliers dont la solubilité dans l'eau est modifiée au-delà d'une certaine température. Il s'agit des polymères présentant une température de demixtion par chauffage (ou point de trouble) définissant ainsi leur zone de solubilité dans l'eau. La température de demixtion minimale obtenue en fonction de la concentration en polymère est appelée "LCST" (Lower Critical Solution Temperature).

Certains de ces polymères sont notamment décrits dans les articles de Taylor et al, Journal of Polymer Science, part A : Polymer Chemistry, 1975, 13, 2551 ; de Bailey et al, Journal of Applied Polymer Science, 1959, 1,56 ; et de Heskins et al, Journal of Macromolecular Science, Chemistry A2, 1968, 1441.

Selon l'enseignement des demandes de brevet EP-A-0583814 et EP-A-0629649, certains polymères ayant une température critique du type LCST sont utilisés comme additifs viscosifiants thermoréversibles dans la fabrication de fluides ou de lubrifiants utilisés dans de nombreux secteurs industriels.

On connaît également par WO98/31643 des polymères en peigne comprenant des motifs oxyalkylénés, et susceptibles d'être employés en émulsion en présence d'agent anti-mousse, dans des compositions de ciment.

On connaît encore par la publication Hourdet et al. "Synthesis of thermoassociative copolymers" Polymer, vol. 38, n°10, PP2535-2547, des polymères à LCST comprenant des motifs oxyalkylénés.

Par ailleurs, il est connu, notamment par la demande WO95/24430 d'employer de tels polymères sensibles à la température et au pH, dans les domaines cosmétique ou pharmaceutique. Les polymères décrits dans cette demande peuvent être de toute nature chimique; en particulier ils peuvent se présenter sous la forme de copolymères greffés comportant un squelette sensible au pH avec des greffons sensibles à la température; ou à l'inverse sous la forme d'un squelette sensible à la température portant des greffons sensibles au pH; ou encore sous la forme de copolymères blocs formés d'unités sensibles au pH et d'unités sensibles à la température.

Les greffons ou blocs sensibles à la température possèdent donc une température de type LCST telle que définie ci-dessus. Ces blocs ou greffons peuvent être préparés par polymérisation de monomères vinyliques ou par polymérisation de monomères éthers cycliques. En particulier ces greffons ou blocs peuvent se présenter sous la forme de poly(N-alkyl substitués) acrylamides ou de copolymères blocs d'oxyde d'éthylène et d'oxyde de propylène.

Toutefois, les propriétés de gélification par chauffage de ces polymères conduisent à des gels opaques, ainsi que cela est précisé dans la description de cette demande de brevet. Or, cette opacification des solutions aqueuses peut être un inconvénient rédhibitoire pour des applications dans le domaine cosmétique.

Il en est de même vis-à-vis de la demande WO97/00275, qui décrit dans de nombreuses applications cosmétiques l'utilisation de polymères ayant des unités thermosensibles, se présentant uniquement sous la forme de copolymères blocs d'oxyde d'éthylène et d'oxyde de propylène. Par ailleurs, dans cette demande, il n'est pas aisé de contrôler la structure et la nature chimique des polymères synthétisés.

La présente invention a pour but de pallier les inconvénients de l'art antérieur et de proposer une nouvelle famille de polymères qui permet de contrôler la rhéologie de compositions aqueuses en fonction de la température, tout en conservant aux compositions une certaine transparence.

Par ailleurs, la structure chimique de cette nouvelle famille de polymères permet d'une part de les préparer à la demande, quelle que soit la nature du polymère squelette et/ou des greffons, et/ou leur quantité respective et/ou la structure recherchée (par exemple linéaire ou branchée); ceci permet notamment d'adapter les propriétés recherchées, en fonction des applications envisagées.

L'objet de la présente invention est une composition aqueuse selon la revendication 1.

Les sels des polymères selon l'invention peuvent être de toutes natures, organiques ou minéraux, par exemple des sels de sodium, de magnésium, d'ammonium, de triéthanolamine,

On obtient ainsi des polymères hydrosolubles, notamment ayant une solubilité dans l'eau, à 20°C, d'au moins 10 g/l, de préférence d'au moins 20 g/l.

Ces polymères hydrosolubles permettent de contrôler la rhéologie de compositions aqueuses en fonction de la température, tout en conservant leur transparence auxdites compositions.

Par ailleurs, il est possible d'ajuster à volonté la gamme de température pertinente pour l'application cosmétique envisagée, en choisissant de manière adéquate la nature chimique des unités hydrosolubles, des unités à LCST, ainsi que leurs quantités respectives.

Les polymères selon la présente invention peuvent être des polymères séquencés (ou blocs), ou des polymères

greffés, qui comprennent d'une part des unités hydrosolubles et d'autre part des unités ayant une température de type LCST telles que définies ci-après.

Les polymères employés dans le cadre de l'invention peuvent donc être des polymères séquencés, comprenant par exemple des séquences hydrosolubles alternées avec des séquences à LCST.

Ces polymères peuvent également se présenter sous la forme de polymères greffés dont le squelette est formé d'unités hydrosolubles, porteur de greffons à LCST. Cette structure peut être partiellement réticulée.

Par unités hydrosolubles, on entend des unités solubles dans l'eau, à 20°C, à raison d'au moins 10 g/l, de préférence au moins 20 g/l.

Toutefois, on peut également employer comme unités hydrosolubles, des unités ne possédant pas obligatoirement la solubilité ci-dessus mentionnée, mais qui en solution à 1% en poids dans de l'eau à 20°C permettent l'obtention d'une solution macroscopiquement homogène et transparente, c'est-à-dire ayant une valeur de transmittance maximum de la lumière, quelque soit la longueur d'onde comprise entre 400 et 800 nm, à travers un échantillon de 1 cm d'épaisseur, d'au moins 80%, de préférence-d'au-moins 85%.

Les unités hydrosolubles peuvent se présenter sous forme de blocs au sein d'un polymère-blocs, ou constituer le squelette d'un polymère greffé.

Ces unités hydrosolubles ne présentent pas de température de démixtion par chauffage de type LCST.

Ces unités hydrosolubles peuvent être obtenues par polymérisation radicalaire de monomères vinyliques, ou par polycondensation, ou encore peuvent être constituées par des polymères naturels ou naturels modifiés existants.

Dans tous les cas, les unités hydrosolubles employées portent au moins deux sites réactifs.

A titre d'exemple, on peut citer les monomères A suivants énumérés ci-après, qui sont susceptibles d'être employés pour former lesdites unités hydrosolubles, seuls ou en mélange, tels quels ou sous la forme de sels organiques ou minéraux, par exemple, sous la forme de sels de sodium, de magnésium, d'ammonium ou de triéthanolamine :

- l'acide (méth)acrylique,
- les monomères vinyliques de formule (IIa) suivante :

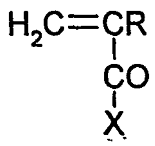

(IIa)

dans laquelle :

- R est choisi parmi H, $-CH_3$, $-C_2H_5$ ou $-C_3H_7$
- X est choisi parmi :
  - les oxydes d'alkyle de type -OR' où R' est un radical hydrocarboné, linéaire ou ramifié, saturé ou insaturé, ayant de 1 à 8 carbones, substitué par au moins un groupement hydroxy (-OH); amine primaire ($-NH_2$); amine secondaire ($-NHR_1$) ou tertiaire ($-NR_1R_2$) avec $R_1$ et $R_2$, indépendamment l'un de l'autre, représentant un radical hydrocarboné, linéaire ou ramifié, saturé ou insaturé ayant 1 à 25 atomes de carbone, sous réserve que-la somme des atomes de carbone de $R_1 + R_2$ ne dépasse pas 26; un atome d'halogène (iode, brome, chlore, fluor);
  - les groupements $-NH_2$, -NHR' et -NR'R" dans lesquels R' et R" sont indépendamment l'un de l'autre des radicaux hydrocarbonés, linéaires ou ramifiés, saturés ou insaturés ayant 1 à 25 atomes de carbone, sous réserve que le nombre total d'atomes de carbone de R' + R" ne dépasse pas 26, lesdits R' et R" étant éventuellement substitués par un groupement hydroxy (-OH); sulfonique ($-SO_3$); sulfate ($-SO_4$); phosphate ($-PO_4H_2$); amine primaire ($-NH_2$); amine secondaire ($-NHR_1$), tertiaire ($-NR_1R_2$) et/ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 1 à 25 atomes de carbone, sous réserve que la somme des atomes de carbone de $R_1 + R_2$ ne dépasse pas 26, et que la somme des atomes de carbone de $R_1 + R_2 + R_3$ ne dépasse pas 27;
- l'anhydride maléique;
- l'acide itaconique;
- l'alcool vinylique de formule $CH_2$=CHOH;

- l'acétate de vinyle de formule $CH_2$=CH-OCOCH3.

Outre les monomères A ci-dessus mentionnés, qui permettent seuls ou en mélange l'obtention d'une unité hydrosoluble ayant au moins deux sites réactifs, il est possible d'employer, en association avec ces monomères A , d'autres monomères B qui ne permettent pas, seuls, l'obtention d'une unité hydrosoluble ayant un site réactif.
Parmi les monomères B, on peut citer, seuls ou en mélange les monomères ci-après, pris tels quels ou sous la forme de sels organiques ou minéraux, par exemple, sous la forme de sels de sodium, de magnésium, d'ammonium ou de triéthanolamine :

- les monomères vinyliques de formule (IIb) suivante :

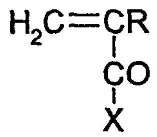

(IIb)

dans laquelle :

- R est choisi parmi H, $-CH_3$, $-C_2H_5$ ou $-C_3H_7$
- X est choisi parmi les oxydes d'alkyle de type -OR' où R' est un radical hydrocarboné, linéaire ou ramifié, saturé ou insaturé, ayant de 1 à 8 carbones, éventuellement substitué par un groupement sulfonique ($-SO_3$), sulfate ($-SO_4$), phosphate ($-PO_4H_2$); et/ou amine quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 1 à 25 atomes de carbone, sous réserve que la somme des atomes de carbone de $R_1 + R_2 + R_3$ ne dépasse pas 27;
- les N-vinyllactames tels que la N-vinylpyrrolidone, la N-vinylcaprolactame et la N-butyrolactame;
- les vinyléthers de formule $CH_2$=CHOR dans laquelle R est un radical hydrocarboné, linéaire ou ramifié, saturé ou insaturé, ayant de 1 à 25 carbones;
- les dérivés hydrosolubles du styrène, notamment le styrène sulfonate;
- le chlorure de diméthyldiallyl ammonium;
- la vinylacétamide.

Parmi les polycondensats et les polymères naturels ou naturels modifiés susceptibles de constituer tout ou partie des unités hydrosolubles, on peut citer :

- les polyuréthanes hydrosolubles ayant au moins deux sites réactifs, notamment portant des fonctions acides carboxyliques,
- la gomme xanthane, notamment celle commercialisée sous les dénominations Keltrol T et Keltrol SF par Kelco; ou Rhodigel SM et Rhodigel 200 de Rhodia;
- les alginates (Kelcosol de Monsanto) et leurs dérivés tels que l'alginate de propylène glycol (Kelcoloid LVF de Kelco);
- les dérivés de cellulose et notamment la carboxyméthylcellulose, l'hydroxypropylcellulose, l'hydroxyéthylcellulose et l'hydroxyéthylcellulose quaternisée;
- les galactomannanes et leurs dérivés, tels que la gomme Konjac, la gomme de guar, l'hydroxypropylguar, l'hydroxypropylguar modifié par des groupements méthylcarboxylate de sodium, le chlorure de guar hydroxypropyl tri-méthyl ammonium.

On peut également citer la polyéthylène imine.
De préférence, les unités hydrosolubles ont une masse molaire comprise entre 10000 g/mole et 5 000 000 g/mole lorsqu'elles constituent le squelette hydrosoluble d'un polymère greffé.
Ces unités hydrosolubles ont de préférence une masse molaire comprise entre 5000 g/mole et 100000 g/mole lorsqu'elles constituent un bloc d'un polymère multiblocs.
Ainsi que cela a été défini plus haut, les unités hydrosolubles portent au moins deux sites réactifs, susceptibles

de réagir avec au moins un site réactif porté par les unités à LCST, afin de donner une liaison covalente.

Ce site réactif peut notamment être choisi parmi les fonctions alcool, isocyanate, amine primaire, secondaire ou tertiaire, acide carboxylique, halogène.

Notamment, un site réactif du type acide carboxylique va généralement réagir avec un site réactif du type alcool ou amine; un site isocyanate va plutôt réagir avec un site alcool, et un site halogène va plutôt réagir avec un site alcool ou amine.

Par unités à LCST, on entend des unités dont la solubilité dans l'eau est modifiée au-delà d'une certaine température. Il s'agit d'unités présentant une température de demixtion par chauffage (ou point de trouble) définissant leur zone de solubilité dans l'eau. La température de demixtion minimale obtenue en fonction de la concentration en polymère est appelée « LCST » (Lower Critical Solution Temperature). Pour chaque concentration en polymère, cette température de demixtion par chauffage est observée; elle est supérieure à la LCST qui est le point minimum de la courbe. En dessous de cette température, le polymère est soluble dans l'eau; au dessus de cette température, le polymère perd sa solubilité dans l'eau.

Par soluble dans l'eau, on entend que les unités présentent une solubilité à 20°C, d'au moins 1 g/l, de préférence au moins 2 g/l.

La mesure de la LCST peut se faire visuellement : on détermine la température à laquelle apparaît le point de trouble de la solution aqueuse; ce point de trouble se traduit par l'opacification de la solution, ou perte de transparence.

D'une manière générale, une composition transparente aura une valeur de transmittance maximum de la lumière, quelque soit la longueur d'onde comprise entre 400 et 800 nm, à travers un échantillon de 1 cm d'épaisseur, d'au moins 80%, de préférence d'au moins 90% (voir EP291334).

La transmittance peut être mesurée en plaçant un échantillon de 1 cm d'épaisseur dans le rayon lumineux d'un spectrophotomètre travaillant dans les longueurs d'onde du spectre lumineux.

Les unités à LCST employées dans la présente invention sont constituées de copolymères statistiques d'oxyde d'éthylène et d'oxyde de propylène, pouvant être représentés par la formule :

$$(OE)_m(OP)_n$$

dans laquelle m est un nombre compris entre 1 et 40 inclus et n est un nombre compris entre 15 et 60 inclus.

De préférence, m est compris entre 1 et 20 inclus et n est compris entre 20 et 50 inclus.

De préférence, la masse molaire des unités à LCST est comprise entre 1500 et 5300 g/mole, notamment comprise entre 2000 et 4000 g/mole.

On a constaté que la répartition statistique des motifs OE et OP se traduit par l'existence d'une température inférieure critique de démixtion, au delà de laquelle une séparation de phases macroscopique est observée.

Ce comportement est différent de celui des copolymères OE OP à blocs, qui micellisent au delà d'une température critique dite de micellisation (agrégation à l'échelle microscopique).

Les unités à LCST doivent bien entendu porter également au moins un site réactif, susceptible de réagir avec le site réactif porté par l'unité hydrosoluble, de manière à former une liaison covalente.

De même que précédemment, ce site réactif peut être choisi parmi les fonctions alcool, isocyanate, amine primaire, secondaire ou tertiaire, acide carboxylique, halogène.

Lorsque le polymère final est constitué d'un squelette hydrosoluble et de greffons à LCST, les sites réactifs sont distribués de façon statistique le long du squelette hydrosoluble et ceux des greffons sont situés à au moins l'une des extrémités des chaînes à LCST.

Lorsque le polymère est de type multiblocs, les sites réactifs sont situés aux extrémités des unités hydrosolubles et des unités à LCST.

Les unités à LCST peuvent donc notamment se présenter sous la forme de copolymères statistiques d'oxyde d'éthylène et d'oxyde de propylène, aminés, notamment monoaminés, diaminés ou triaminés.

Parmi les unités à LCST commercialement disponibles, on peut citer les copolymères vendus sous la dénomination Jeffamine par HUNTSMAN, et notamment la Jeffamine XTJ-507 (M-2005), la Jeffamine D-2000 et la Jeffamine XTJ-509 (ou T-3000).

Les unités à LCST peuvent également se présenter sous la forme de polyalkylènes glycols, copolymères OE OP statistiques à extrémités OH, tels que ceux vendus sous la dénomination Polyglycols P41 et B11 par Clariant.

Parmi les polymères finals susceptibles d'être employés dans le cadre de l'invention, on peut en particulier citer :

- les polymères dont le squelette est constitué par :

  - un homopolymère linéaire d'acide acrylique,
  - un copolymère linéaire d'acide acrylique et d'AMPS, et/ou d'acrylamide,
  - un homopolymère réticulé d'acide polyacrylique,

- un dérivé naturel tel que le Xanthane, les alginates, la carboxyméthylcellulose, l'hydroxypropylguar modifié par des groupements méthylcarboxylate de sodium;

ou l'un de leurs sels organiques ou minéraux ;

- et portant des greffons à LCST constitués par des copolymères statistiques d'oxyde d'éthylène et d'oxyde de propylène, aminés, notamment monoaminés, diaminés ou triaminés.

La proportion massique des unités à LCST dans le polymère final est de préférence comprise entre 5% et 70%, notamment entre 20% et 65%, et particulièrement entre 30% et 60% en poids, par rapport au polymère final.

De préférence, la température de demixtion par chauffage de type LCST desdites unités à LCST est comprise entre 5°C et 40°C, de préférence 10°C et 35°C, pour une concentration massique dans l'eau, de 1 % en poids desdites unités à LCST.

Les polymères employés dans le cadre de l'invention peuvent être aisément préparés par l'homme du métier sur la base de ses connaissances générales.

Notamment, lorsque le polymère final se présente sous la forme d'un polymère greffé notamment présentant un squelette hydrosoluble avec des unités à LCST greffées, il est possible de le préparer par greffage des chaînes à LCST ayant au moins une extrémité réactive, notamment aminée, sur ledit polymère hydrosoluble formant le squelette, portant au minimum 10% (en mole) de groupes acides carboxyliques.

Cette réaction peut s'effectuer en présence d'un carbodiimide tel que le dicyclohexylcarbodiimide ou le chlorhydrate de 1-(3-diméthylaminopropyl)-3-éthyl carbodiimide, dans un solvant tel que la N-méthylpyrrolidone ou l'eau.

Pour ce faire, on peut s'inspirer du mode de préparation décrit dans la publication Hourdet et al., Polymer, vol. 38, no10, pp.2535-2547, 1997.

Cette publication décrit notamment la préparation de polymères thermoassociatifs comprenant un squelette hydrosoluble acide polyacrylique, sur lequel sont greffés des unités à LCST constituées de copolymères statistiques d'oxyde d'éthylène et d'oxyde de propylène. Toutefois, les polymères décrits dans cette publication ne conviennent pas dans la présente application car ils présentent certains inconvénients notamment liés à la faible masse molaire des greffons employés; notamment le greffon appelé PPO présente une masse molaire de 600.

En effet, lorsque la masse molaire des greffons est trop faible, la température de gélification du polymère final ne correspond pas tout-à-fait à la température de démixtion des greffons à LCST; il peut exister entre ces deux températures un décalage pouvant atteindre 15 à 20°C. La prédiction des propriétés de thermogélification en vue d'application industrielle devient alors complexe.

Par ailleurs, la quantité d'unités à LCST à incorporer dans le polymère pour avoir des propriétés en solution intéressantes implique des taux de greffage élevés lorsque la masse molaire du greffon est peu importante. Ceci impose notamment la présence d'une proportion importante de sites réactifs sur le squelette hydrosoluble, d'où une certaine limitation en terme de structure et de synthèse.

Par ailleurs, l'association des greffons peut devenir plus difficile lorsqu'ils sont de taille plus petite.

Une autre possibilité pour préparer des polymères greffés consiste à copolymérisser par exemple un macromonomère à LCST (chaîne à LCST précédemment décrite avec une extrémité vinylique) et un monomère vinylique hydrosoluble tel que l'acide acrylique ou les monomères vinyliques ayant la formule (IIa) ou (IIb).

Lorsque le polymère final se présente sous la forme d'un polymère à blocs, il est possible de le préparer par couplage entre des unités hydrosolubles et des unités à LCST ayant à chaque extrémité des sites réactifs complémentaires.

Les polymères ainsi obtenus sont hydrosolubles et thermogélifiants.

Ils permettent l'obtention de compositions aqueuses épaissies, voire gélifiées, qui présentent notamment une viscosité constante ou qui augmente lorsque la température augmente, et qui présentent par ailleurs une bonne transparence.

Dans le cadre de la présente invention, on entend par solution ou composition transparente, la définition classique donnée dans le dictionnaire. Ainsi, une composition transparente se laisse aisément traverser par la lumière et permet de distinguer nettement les objets à travers son épaisseur.

La transmittance peut être mesurée en plaçant un échantillon de 1 cm d'épaisseur dans le rayon lumineux d'un spectrophotomètre travaillant dans les longueurs d'onde du spectre lumineux.

Notamment, les compositions ainsi préparées peuvent avoir une valeur de transmittance maximum de la lumière, quelque soit la longueur d'onde comprise entre 400 et 800 nm, à travers un échantillon de 1 cm d'épaisseur, d'au moins 80%, de préférence d'au moins 85% (voir EP291334).

Les polymères selon l'invention sont de préférence présents dans les compositions aqueuses en une quantité de préférence comprise entre 0,01 et 20% en poids, notamment de 0,05 à 15% en poids, et en particulier de 0,1 à 10% en poids.

Ces compositions et les polymères qu'elles comprennent trouvent une application toute particulière dans les

domaines cosmétique et pharmaceutique.

Ladite composition comprend, outre le polymère tel que ci-dessus défini, une phase aqueuse, qui peut comprendre, outre de l'eau, une eau florale telle que l'eau de bleuet, une eau minérale telle que l'eau de VITTEL, l'eau de LUCAS ou l'eau de LA ROCHE POSAY et/ou une eau thermale.

Les compositions selon l'invention peuvent contenir une phase huileuse, par exemple, sous la forme d'une émulsion huile-dans-eau, d'une émulsion eau-dans-huile ou d'émulsions multiples telles que les émulsions eau-dans-huile-dans-eau.

Il est possible d'ajouter dans ladite composition aqueuse les constituants usuellement utilisés dans le type d'application envisagé. Bien entendu l'homme du métier veillera à choisir ces éventuels constituants complémentaires, et/ou leur quantité, de manière telle que les propriétés avantageuses de la composition selon l'invention ne soient pas, ou substantiellement pas, altérées par l'adjonction envisagée.

Ladite composition aqueuse peut former tout ou partie d'une composition cosmétique ou pharmaceutique qui peut donc comprendre par ailleurs un milieu cosmétiquement ou pharmaceutiquement acceptable, c'est-à-dire un milieu compatible avec une application sur les matières kératiniques telles que la peau, les ongles, les cheveux, les cils et sourcils, les muqueuses et les semi-muqueuses, et toute autre zone cutanée du corps et du visage.

Ladite composition trouve donc une application particulière comme composition cosmétique, de maquillage ou de soin, susceptible d'être appliquée sur la peau, les ongles, les cheveux, les cils et sourcils, les muqueuses et les semi-muqueuses, et toute autre zone cutanée du corps et du visage.

L'invention est illustrée plus en détail dans les exemples suivants.

## Exemple 1

Dans un réacteur de 500 ml muni d'un réfrigérant, on dissout 3 grammes d'acide polyacrylique de masse molaire moyenne 450 000 g/mole (Aldrich) dans 220 ml de N-méthyl pyrrolidone sous agitation à 60°C pendant 12 heures.

On dissout 4,181 grammes de copolymère $(OE)_6(OP)_{39}$ statistique mono-aminé, de masse molaire 2600 g/mole ayant un point de trouble, à une concentration de 1% en poids dans l'eau, de 16°C (Jeffamine M-2005 de Huntsman) dans 50 ml de N-méthylpyrrolidone sous agitation, à 20°C, pendant 15 minutes. La solution obtenue est ajoutée goutte à goutte au milieu réactionnel contenant l'acide polyacrylique, sous vive agitation à 60°C.

On dissout 2,158 grammes de dicyclohexylcarbodiimide dans 30 ml de N-méthylpyrrolidone sous agitation à 20°C pendant 15 minutes. La solution obtenue est ajoutée goutte à goutte au milieu réactionnel contenant l'acide polyacrylique et le copolymère $(OE)_6(OP)_{39}$ statistique mono-aminé, sous vive agitation à 60°C.

On agite le mélange final pendant 12 heures à 60°C.

On refroidit le mélange à 20°C puis on le place dans un réfrigérateur à 4°C pendant 24 heures.

Les cristaux de dicyclohexylurée formés sont éliminés par filtration du milieu réactionnel.

Le polymère est alors neutralisé à l'aide de 19 g de soude à 35% (4 fois en excès par rapport au nombre de mole d'acide acrylique), ce qui conduit à sa précipitation. Après 12 heures au repos, le milieu réactionnel est filtré afin de récupérer le polymère précipité. Celui-ci est séché sous vide à 35°C pendant 24 heures.

On récupère 13,55 grammes de solide qui sont dissous dans 2 litres d'eau désionisée. Cette solution est ultrafiltrée à l'aide d'un système d'ultrafiltration Millipore contenant une membrane dont le seuil de coupure est fixée à 10 000 Daltons. La solution ainsi purifiée est lyophilisée afin de recueillir le polymère sous forme solide.

On obtient 7,05 grammes de polyacrylate de sodium (450 000 g/mole) greffé par 3,9% (en mole) de copolymère $(OE)_6(OP)_{39}$ statistique mono-aminé.

La proportion massiques des unités à LCST dans le polymère final est de 51 %.

Le polymère ainsi obtenu présente une solubilité dans l'eau, à 20°C, d'au moins 10 g/l.

## Exemple 2

De manière similaire à l'exemple 1, on prépare les cinq polymères suivants, dans lesquels les greffons sont toujours un copolymère $(OE)_6(OP)_{39}$ statistique mono-aminé :

| | Squelette | Proportion massique des unités à LCST dans le polymère final | Taux de greffage (% en mole) |
|---|---|---|---|
| Exemple 2a | polyacrylate de sodium (PM: 450000) | 35% | 2% |
| Exemple 2b | polyacrylate de sodium (PM: 750000) | 58% | 5,2% |

(suite)

| | Squelette | Proportion massique des unités à LCST dans le polymère final | Taux de greffage (% en mole) |
|---|---|---|---|
| Exemple 2c | polyacrylate de sodium (PM: 750000) | 51% | 3,9% |
| Exemple 2d | polyacrylate de sodium (PM: 450000) | 59% | 5,3% |
| Exemple 2e | Carbomer (Carbopol 980 de Goodrich) Sous forme de sel de sodium | 51% | 3,9% |

**Exemple 3**

Dans un réacteur de 1 litre muni d'un réfrigérant, on dissout 1,51 grammes d'acide polyacrylique de masse molaire moyenne 750 000 g/mole (Aldrich) dans 350 ml d'eau désionisée sous agitation à 20°C pendant 12 heures. Le pH du milieu réactionnel est alors ajusté à 8 à l'aide d'une solution de soude 1 M.

On dissout 1,60 grammes de copolymère $(OE)_6(OP)_{39}$ statistique mono-aminé (Jeffamine M-2005 de Huntsman) dans 100 ml d'eau désionisée sous agitation à 5°C pendant 30 minutes. La solution obtenue est ajoutée goutte à goutte au milieu réactionnel sous vive agitation.

On dissout 1,84 grammes de chlorhydrate de 1-(3-diméthylaminopropyl)-3-éthyl carbodiimide dans 50 ml d'eau désionisée sous agitation à 20°C pendant 15 minutes. La solution obtenue est ajoutée sous vive agitation, goutte à goutte, au milieu réactionnel précédent qui est alors chauffé à 60°C pendant 6 heures.

Le milieu réactionnel est refroidi à 20°C puis concentré et précipité dans l'acétone. Le polymère sous forme solide est récupéré par filtration et lavé avec un excès d'acétone. La poudre est broyée et séchée sous vide à 35°C.

On obtient 3,33 g de polyacrylate de sodium (750 000 g/mole) greffé par 3,1 % (en mole) de copolymère $(OE)_6(OP)_{39}$ statistique mono-aminé (rendement 94%).

**Exemple 4**

On compare les polymères selon l'invention avec les polymères de l'art antérieur décrits dans la publication Hourdet et al., Polymer, vol.38, no10, pp.2535-2547, 1997.

Composés testés :

| | Squelette hydrosoluble | Greffons (unités à LCST) | Proportion : unités à LCST dans polymère final (en poids) | Taux de greffage (% en mole) |
|---|---|---|---|---|
| Exemple 1 | polyacrylate de sodium; PM=450000 | $(OE)_6(OP)_{39}$ statistique monoaminé; PM=2600 | 51% | 3,9% |
| Exemple 2a | polyacrylate de sodium; PM=450000 | $(OE)_6(OP)_{39}$ statistique monoaminé; PM=2600 | 35% | 2% |
| Exemple 2d | polyacrylate de sodium; PM=450000 | $(OE)_6(OP)_{39}$ statistique monoaminé; PM=2600 | 59% | 5,3% |

(suite)

| | Squelette hydrosoluble | Greffons (unités à LCST) | Proportion : unités à LCST dans polymère final (en poids) | Taux de greffage (% en mole) |
|---|---|---|---|---|
| Comparatif 1 | polyacrylate de sodium; PM=150000 | $(OE)_1(OP)_9$ statistique monoaminé; PM=600 | 30% | 7% |
| Comparatif 2 | polyacrylate de sodium; PM=150000 | $(OE)_1(OP)_9$ statistique monoaminé; PM=600 | 55% | 21% |

On détermine, pour chacun de ces composés, à une concentration donnée dans l'eau, la différence entre la température Tgel de gélification de la solution aqueuse de polymère, et la température Tdem de démixtion des greffons à LCST seuls.

$$\Delta T = Tgel - Tdem$$

La température de gélification est déterminée à l'aide des courbes rhéologiques mesurant la viscosité en fonction de la température, pour la solution de polymère d'une part et pour une solution aqueuse de squelette hydrosoluble à une concentration identique à la concentration en unités hydrosolubles dans la solution de polymère.
On considère la température de gélification atteinte lorsque la différence entre la viscosité de la solution de polymère et la viscosité de la solution de squelette hydrosoluble, est supérieure à 5%. La méthode de mesure de la viscosité est donnée dans l'exemple 6.
La température de démixtion est déterminée visuellement, pour une solution en greffons à LCST seuls, à une concentration identique à la concentration des greffons dans la solution de polymère.
On considère que la température de démixtion est atteinte lorsque la solution blanchit, c'est-à-dire n'est plus transparente au sens de la présente invention.

| Polymère | Concentration dans l'eau (% en poids) | Tgel | Tdem | ΔT |
|---|---|---|---|---|
| Exemple 1 | 2% | 28 | 23 | 5 |
| Exemple 1 | 5% | 23 | 19 | 4 |
| Exemple 2a | 2% | 33 | 27 | 6 |
| Exemple 2d | 5% | 22 | 19 | 3 |
| Comparatif 1 | 2% | 65 | 49 | 16 |
| Comparatif 1 | 5% | 63 | 46 | 17 |
| Comparatif 2 | 2% | 30 | 47 | 13 |
| Comparatif 2 | 5% | 53 | 41 | 12 |

On constate donc un bon accord entre la température de démixtion des greffons à LCST et les propriétés du polymère greffé, pour les polymères de l'invention; ceci n'est pas le cas pour les polymères de l'art antérieur.

## Exemple 5

On compare les polymères selon l'invention avec des polymères de l'art antérieur décrits dans WO95/24430.
On mesure, par spectroscopie UV visible, à une longueur d'onde égale à 500 nm, l'absorbance de solutions aqueuses comprenant ces polymères, à une température de 35°C et de 40°C.
On en déduit la transmittance selon la relation : absorbance = -log transmittance.
On obtient les résultats suivants :

| | Transmittance | |
|---|---|---|
| | à 35°C | à 40°C |
| Comparaison 1 à 0,2% en poids dans l'eau | 76% | 63% |
| Comparaison 2 à 0,2% en poids dans l'eau | 50% | 42% |
| Polymère de l'exemple 1 à 5% en poids dans l'eau | 89% | 88% |
| Polymère de l'exemple 2a à 2% en poids dans l'eau | 88% | 87% |

Comparaison 1 : copolymère bloc poly(N-isopropylacrylamide) et acide polyacrylique 24/76 (figure 1 de WO95/24430)

Comparaison 2 : copolymère bloc poly(N-isopropylacrylamide) et acide polyacrylique 43/57 (figure 1 de WO95/24430)

On constate donc que le polymère selon l'invention conduit à des compositions nettement plus transparentes que celles de l'art antérieur.

**Exemple 6**

On prépare un gel aqueux thermogélifiant comprenant :

- polymère de l'exemple 1 (matière sèche) 5 g
- chlorure de sodium 1,17 g
- eau qsp 100 g

Cette composition est préparée par simple introduction du polymère dans l'eau salée sous agitation pendant 2 heures à 20°C.

On mesure la viscosité de la composition, à 20°C et à 32°C, à l'aide d'un rhéomètre Haake RS150 muni d'une géométrie cône/plan 3,5 cm/2° ou 6cm/2° et d'un système de contrôle de la température. Les mesures de viscosité sont réalisées dans le mode écoulement en imposant un gradient de cisaillement égal à 10 $s^{-1}$.

On obtient les résultats suivants :

- viscosité à 20°C : 0,015 Pa.s
- viscosité à 32°C : 5 Pa.s

**Revendications**

**1.** Composition aqueuse comprenant une phase aqueuse et au moins un polymère comprenant des unités hydrosolubles portant au moins deux sites réactifs, et des unités ayant une température de type LCST portant au moins un site réactif, susceptible de réagir avec les sites réactifs portés par l'unité hydrosoluble, de manière à former une liaison covalente, lesdites unités à LCST étant constituées de copolymères statistiques d'oxyde d'éthylène et d'oxyde de propylène, de formule $(OE)_m(OP)_n$ ayant un nombre d'oxyde d'éthylène (m) compris entre 1 et 40 inclus et un nombre d'oxyde de propylène (n) compris entre 15 et 60 inclus, ainsi que les sels de ce polymère; ledit polymère étant présent en une quantité comprise entre 0,01 et 20% en poids.

**2.** Composition selon la revendication 1, dans laquelle le polymère se présente sous la forme d'un polymères séquencé (ou blocs) comprenant des séquences hydrosolubles alternées avec des séquences à LCST, ou sous la forme d'un polymère greffé dont le squelette est formé d'unités, hydrosolubles et est porteur de greffons à LCST, cette structure pouvant être partiellement; réticulée.

**3.** Composition selon l'une des revendications précédentes, dans laquelle les unités hydrosolubles sont obtenues par polymérisation radicalaire d'au moins un monomère A, pris tel quel ou sous la forme de sel organique ou minéral, ces monomères A étant choisis parmi, seuls ou en mélange:

- l'acide (méth)acrylique,
- les monomères vinyliques de formule (IIa) suivante :

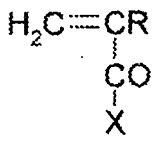

(IIa)

dans laquelle :
- R est choisi parmi H, $-CH_3$, $-C_2H_5$ ou $-C_3H_7$
- X est choisi parmi :

  - les oxydes d'alkyle de type -OR' où R' est un radical hydrocarboné, linéaire ou ramifié, saturé ou insaturé, ayant de 1 à 8 carbones, substitué par au moins un groupement hydroxy (-OH); amine primaire ($-NH_2$); amine secondaire ($-NHR_1$) ou tertiaire ($-NR_1R_2$) avec $R_1$ et $R_2$, indépendamment l'un de l'autre, représentant un radical hydrocarboné, linéaire ou ramifié, saturé ou insaturé ayant 1 à 25 atomes de carbone, sous réserve que la somme des atomes de carbone de $R_1 + R_2$ ne dépasse pas 26; un atome d'halogène (iode, brome, chlore, fluor);
  - les groupements $-NH_2$, -NHR' et -NR'R" dans lesquels R' et R" sont indépendamment l'un de l'autre des radicaux hydrocarbonés, linéaires ou ramifiés, saturés ou insaturés ayant 1 à 25 atomes de carbone, sous réserve que le nombre total d'atomes de carbone de R' + R" ne dépasse pas 26, lesdits R' et R" étant éventuellement substitués par un groupement hydroxy (-OH); sulfonique ($-SO_3$); sulfate ($-SO_4$); phosphate ($-PO_4H_2$); amine primaire ($-NH_2$), amine secondaire ($-NHR_1$), tertiaire ($-NR_1R_2$) et/ou quaternaire ($-N^+R_1R_2R_3$) avec $R_1$, $R_2$ et $R_3$ étant, indépendamment l'un de l'autre, un radical hydrocarboné, linéaires ou ramifié, saturé ou insaturé ayant 1 à 25 atomes de carbone, sous réserve que la somme des atomes de carbone de $R_1 + R_2$ ne dépasse pas 26, et que la somme des atomes de carbone de $R_1 + R_2 + R_3$ ne dépasse pas 27;

- l'anhydride maléique;
- l'acide itaconique;
- l'alcool vinylique de formule $CH_2$=CHOH;
- l'acétate de vinyle de formule $CH_2$=CH-OCOCH3

**4.** Composition selon la revendication 3, dans laquelle les unités hydrosolubles sont obtenues par polymérisation en outre d'au moins un monomère B, pris tel quel ou sous la forme de sel organique ou minéral, seuls ou en mélange,

- les monomères vinyliques de formule (IIb) suivante :

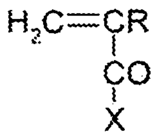

(IIb)

dans laquelle:
- R est choisi parmi H, $-CH_3$, $-C_2H_5$ ou $-C_3H_7$
- X est choisi parmi les oxydes d'alkyle de type -OR' où R' est un radical hydrocarbone, linéaire ou ramifié, saturé ou insaturé, ayant de 1 a 8 carbones, éventuellement substitué par un groupement sulfonique ($-SO_3$), sulfate ($-SO_4$), phosphate ($-PO_4H_2$; et/ou amine quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 1 à 25 atomes de carbone, sous réserve que la somme des atomes de carbone de $R_1 + R_2 + R_3$ ne dépasse pas 27;
- les N-vinyllactames tels que la N-vinylpyrrolidone, la N-vinylcaprolactame et la N-butyrolactame;
- les vinyléthers de formule $CH_2$=CHOR dans laquelle R est un radical hydrocarbone, linéaire ou ramifié, saturé ou insaturé, ayant de 1 a 25 carbones;
- les dérivés hydrosolubles du styrène, notamment le styrène sulfonate;
- le chlorure de diméthyldiallyl ammonium;
- la vinylacétamide.

**5.** Composition selon l'une des revendications 1 à 2, dans laquelle les unités hydrosolubles sont choisies parmi :

- les polyuréthanes hydrosolubles ayant au moins deux sites réactifs, notamment portant des fonctions acides carboxyliques,
- la gomme xanthane,
- les alginates et leurs dérivés tels que l'alginate de propylène glycol;
- les dérivés de cellulose et notamment la carboxyméthylcellulose, l'hydroxypropylcellulose, l'hydroxyéthylcellulose et l'hydroxyéthylcellulose quaternisée;
- les galactomannanes et leurs dérivés, tels que la gomme Konjac, la gomme de guar, l'hydroxypropylguar, l'hydroxypropylguar modifié par des groupements méthylcarboxylate de sodium, le chlorure de guar hydroxypropyl tri-méthyl ammonium:
- la polyéthylène imine.

**6.** Composition selon l'une des revendications précédentes, dans laquelle les unités hydrosolubles ont une masse molaire comprise entre 50000 g/mole et 5 000 000 gazole lorsqu'elles constituent le squelette hydrosoluble d'un polymère greffé; ou une masse molaire comprise entre 5000 gimole et 100000 g/mole lorsqu'elles constituent un bloc d'un polymère multiblocs.

**7.** Composition selon l'une des revendications précédentes, dans laquelle les unités à LCST sont constituées de copolymères statistiques d'oxyde d'éthylène et d'oxyde de propylène, de formule $(OE)_m(OP)_n$ dans laquelle m est un nombre compris entre 1 et 20 inclus et n est un nombre compris entre 20 et 50 inclus.

**8.** Composition selon l'une des revendications précédentes, dans laquelle la masse molaire des unités à LCST est comprise entre 1500 et 5300 g/mole, notamment comprise entre 2000 et 4000 g/mole.

**9.** Composition selon l'une des revendications précédentes, dans laquelle les unités à LCST se présentent sous la forme de copolymères statistiques d'oxyde d'éthylène et d'oxyde de propylène, aminés, notamment monoamines, diaminés ou triaminés.

**10.** Composition selon l'une des revendications précédentes, dans laquelle la proportion massique des unités à LCST dans le polymère final est comprise entre 5% et 70%, notamment entre 20% et 65%, et particulièrement entre 30% et 60% en poids, par rapport au polymère final.

**11.** Composition selon l'une des revendications précédentes, dans laquelle la température de demixtion par chauffage de type LCST desdites unités à LCST est comprise entre 5°C et 40°C, de préférence 10°C et 35°C, pour une concentration massique dans l'eau, de 1% en poids desdites unités à LCST.

**12.** Composition selon l'une des revendications précédentes, dans laquelle le polymère présente une solubilité dans l'eau, à 20°C, d'au moins 10 g/l, de préférence d'au moins 20 g/l.

**13.** Composition selon l'une des revendications précédentes, se présentant sous forme d'une composition aqueuse épaissie, voire gélifiée, qui présente une valeur de transmittance maximum de la lumière, quelque soit la longueurs d'onde comprise entre 400 et 800 nm, à travers un échantillon de 1 cm d'épaisseur, d'au moins 80%, de préférence d'au moins 85%.

**14.** Composition selon l'une des revendications précédentes, dans laquelle les polymères sont présents en une quantité comprise entre 0,05 et 15% en poids, et en particulier de 0,1 à 10% en poids.

**15.** Composition selon l'une des revendications précédentes, comprenant par ailleurs un milieu cosmétiquement ou pharmaceutiquement acceptable.

**16.** Composition selon l'une des revendications précédentes, se présentant sous la forme d'une composition cosmétique, de maquillage ou de soin, susceptible d'être appliquée sur la peau, les ongles, les cheveux, les cils et sourcils, les muqueuses et les semi-muqueuses, et toute autre zone cutanée du corps et du visage.

## Claims

**1.** Aqueous composition comprising an aqueous phase and at least one polymer comprising water-soluble units bearing at least two reactive sites, and units having a temperature of LCST type bearing at least one reactive site, capable

of reacting with the reactive sites borne by the water-soluble unit, so as to form a covalent bond, said units with an LCST consisting of random copolymers of ethylene oxide and propylene oxide, of the formula $(EO)_m(PO)_n$, having an ethylene oxide number (n) of between 1 and 40 inclusive and a propylene oxide number (n) of between 15 and 60 inclusive, and the salts of this polymer; said polymer being present in an amount of between 0.01% and 20% by weight.

**2.** Composition according to Claim 1, in which the polymer is in the form of a block polymer comprising water-soluble blocks alternating with blocks with an LCST, or in the form of a grafted polymer whose backbone is formed from water-soluble units and bears grafts with an LCST, this structure possibly being partially crosslinked.

**3.** Composition according to either of the preceding claims, in which the water-soluble units are obtained by free-radical polymerization of at least one monomer A, taken as it is or in the form of an organic or inorganic salt, these monomers A being chosen, alone or as a mixture, from:

- (meth)acrylic acid,
- vinyl monomers of formula (IIa) below:

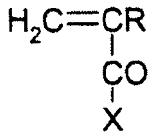

(IIa)

in which:
- R is chosen from H, $-CH_3$, $-C_2H_5$ or $-C_3H_7$;
- X is chosen from:

  - alkyl oxides of -OR' type in which R' is a linear or branched, saturated or unsaturated hydrocarbon-based radical containing from 1 to 8 carbons, substituted with at least one hydroxyl (-OH); primary amine ($-NH_2$); secondary amine ($-NHR_1$) or tertiary amine ($-NR_1R_2$) group, with $R_1$ and $R_2$, independently of each other, representing a linear or branched, saturated or unsaturated hydrocarbon-based radical containing 1 to 25 carbon atoms, with the proviso that the sum of the carbon atoms of $R_1 + R_2$ does not exceed 26; a halogen atom (iodine, bromine, chlorine or fluorine);
  - groups $-NH_2$, -NHR' and -NR'R" in which R' and R" are, independently of each other, linear or branched, saturated or unsaturated hydrocarbon-based radicals containing 1 to 25 carbon atoms, with the proviso that the total number of carbon atoms of R' + R" does not exceed 26, said R' and R" optionally being substituted with a hydroxyl (-OH); sulfonic ($-SO_3$); sulfate ($-SO_4$) ; phosphate ($-PO_4H_2$) ; primary amine ($-NH_2$) ; secondary amine ($-NHR_1$), tertiary amine ($-NR_1R_2$) and/or quaternary amine ($-N^+R_1R_2R_3$) group, with $R_1$, $R_2$ and $R_3$ being, independently of each other, a linear or branched, saturated or unsaturated hydrocarbon-based radical containing 1 to 25 carbon atoms, with the proviso that the sum of the carbon atoms of $R_1 + R_2$ does not exceed 26, and that the sum of the carbon atoms of $R_1 + R_2 + R_3$ does not exceed 27;

- maleic anhydride;
- itaconic acid;
- vinyl alcohol of formula $CH_2$=CHOH;
- vinyl acetate of formula $CH_2$=CH-$OCOCH_3$.

**4.** Composition according to Claim 3, in which the water-soluble units are obtained by polymerization also of at least one monomer B, taken as it is or in the form of an organic or inorganic salt, these monomers B being chosen, alone or as a mixture, from

- vinyl monomers of formula (IIb) below:

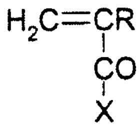 (IIb)

in which:

- R is chosen from H, $-CH_3$, $-C_2H_5$ or $-C_3H_7$;
- X is chosen from alkyl oxides of -OR' type in which R' is a linear or branched, saturated or unsaturated hydrocarbon-based radical containing from 1 to 8 carbons, optionally substituted with a sulfonic ($-SO_3$), sulfate ($-SO_4$), phosphate ($-PO_4H_2$); and/or quaternary amine ($-N^+R_1R_2R_3$) group, with $R_1$, $R_2$ and $R_3$ being, independently of each other, a linear or branched, saturated or unsaturated hydrocarbon-based radical containing 1 to 25 carbon atoms, with the proviso that the sum of the carbon atoms of $R_1 + R_2 + R_3$ does not exceed 27;
- N-vinyllactams such as N-vinylpyrrolidone, N-vinylcaprolactam and N-butyrolactam;
- vinyl ethers of formula $CH_2$=CHOR in which R is a linear or branched, saturated or unsaturated hydrocarbon-based radical containing from 1 to 25 carbons;
- water-soluble derivatives of styrene, especially styrene sulfonate;
- dimethyldiallylammonium chloride;
- vinylacetamide.

**5.** Composition according to either of Claims 1 and 2, in which the water-soluble units are chosen from:

- water-soluble polyurethanes having at least two reactive sites, especially bearing carboxylic acid functions;
- xanthan gum;
- alginates and derivatives thereof such as propylene glycol alginate;
- cellulose derivatives and especially carboxymethylcellulose, hydroxypropylcellulose, hydroxyethylcellulose and quaternized hydroxyethylcellulose;
- galactomannans and derivatives thereof, such as Konjac gum, guar gum, hydroxypropylguar, hydroxypropylguar modified with sodium methylcarboxylate groups, and hydroxypropyltrimethylammonium guar chloride;
- polyethyleneimine.

**6.** Composition according to one of the preceding claims, in which the water-soluble units have a molar mass of between 50 000 g/mol and 5 000 000 g/mol when they constitute the water-soluble backbone of a grafted polymer; or a molar mass of between 5 000 g/mol and 100 000 g/mol when they constitute a block of a multiblock polymer.

**7.** Composition according to one of the preceding claims, in which the units with an LCST consist of random copolymers of ethylene oxide and propylene oxide, of the formula $(EO)_m(PO)_n$ in which m is a number between 1 and 20 inclusive and n is a number between 20 and 50 inclusive.

**8.** Composition according to one of the preceding claims, in which the molar mass of the units with an LCST is between 1 500 and 5 300 g/mol and especially between 2 000 and 4 000 g/mol.

**9.** Composition according to one of the preceding claims, in which the units with an LCST are in the form of random copolymers of ethylene oxide and propylene oxide, aminated, in particular monoaminated, diaminated or triaminated.

**10.** Composition according to one of the preceding claims, in which the proportion by mass of the units with an LCST in the final polymer is between 5% and 70%, especially between 20% and 65%, and particularly between 30% and 60% by weight relative to the final polymer.

**11.** Composition according to one of the preceding claims, in which the heat-induced demixing temperature of LCST type of said units with an LCST is between 5°C and 40°C and preferably 10°C and 35°C, for a concentration by mass in water of 1% by weight of said units with an LCST.

**12.** Composition according to one of the preceding claims, in which the polymer has a solubility in water, at 20°C, of at least 10 g/l and preferably of at least 20 g/l.

**13.** Composition according to one of the preceding claims, which is in the form of a thickened, or even gelled, aqueous composition, which has a maximum light transmittance value, irrespective of the wavelength of between 400 and

800 nm, through a sample 1 cm thick, of at least 80% and preferably of at least 85%.

**14.** Composition according to one of the preceding claims, in which the polymers are present in an amount of between 0.05% and 15% by weight and in particular from 0.1% to 10% by weight.

**15.** Composition according to one of the preceding claims, moreover comprising a cosmetically or pharmaceutically acceptable medium.

**16.** Composition according to one of the preceding claims, provided in the form of a cosmetic, make-up or care composition, which may be applied to the skin, the nails, the hair, the eyelashes, the eyebrows, the mucous membranes and semi-mucous membranes, and any other area of body or facial skin.

**Patentansprüche**

**1.** Wässrige Zusammensetzung, umfassend eine wässrige Phase und mindestens ein Polymer mit wasserlöslichen Einheiten mit mindestens zwei reaktiven Stellen und Einheiten mit einer Temperatur vom LCST-Typ mit mindestens einer reaktiven Stelle, die mit den reaktiven Stellen der wasserlöslichen Einheit unter Bildung einer kovalenten Bindung reagieren kann, wobei die Einheiten mit LCST aus statistisch aufgebauten Copolymeren von Ethylenoxid und Propylenoxid der Formel $(EO)_m(PO)_n$ mit einer Ethylenoxid-Zahl (m) zwischen 1 und 40 inklusive und einer Propylenoxid-Zahl (n) zwischen 15 und 60 inklusive bestehen, sowie die Salze dieses Polymers; wobei das Polymer in einer Menge zwischen 0,01 und 20 Gew.-% vorliegt.

**2.** Zusammensetzung nach Anspruch 1, wobei das Polymer in Form eines Blockpolymers mit wasserlöslichen Blöcken, die mit Blöcken mit LCST alternieren, oder in Form eines Pfropfpolymers, dessen Hauptkette aus wasserlöslichen Einheiten gebildet ist und Pfropfanteile mit LCST trägt, wobei diese Struktur teilweise vernetzt sein kann, vorliegt.

**3.** Zusammensetzung nach einem der vorhergehenden Ansprüche, wobei die wasserlöslichen Einheiten durch radikalische Polymerisation von mindestens einem Monomer A als solchem oder in Form eines organischen oder anorganischen Salzes erhalten werden, wobei die Monomere A allein oder als Mischung unter:

- (Meth)acrylsäure,
- Vinylmonomeren der folgenden Formel (IIa):

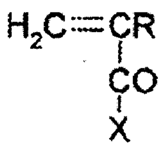

(IIa)

worin:
- R unter H, $-CH_3$, $-C_2H_5$ oder $-C_3H_7$ ausgewählt ist,
- X unter:

  - Alkyloxiden vom Typ -OR', wobei R' für einen linearen oder verzweigten, gesättigten oder ungesättigten Kohlenwasserstoffrest mit 1 bis 8 Kohlenstoffatomen steht, der durch mindestens eine Hydroxygruppe (-OH); primäre Amingruppe ($-NH_2$); sekundäre Amingruppe ($-NHR_1$) oder tertiäre Amingruppe ($-NR_1R_2$) substituiert ist, wobei $R_1$ und $R_2$ unabhängig voneinander für einen linearen oder verzweigten, gesättigten oder ungesättigten Kohlenwasserstoffrest mit 1 bis 25 Kohlenstoffatomen stehen, mit der Maßgabe, dass die Summe der Kohlenstoffatome von $R_1$ + $R_2$ nicht über 26 hinausgeht; einem Halogenatom (Iod, Brom, Chlor, Fluor);
  - $-NH_2$-, -NHR'- und -NR'R''-Gruppen, worin R' und R" unabhängig voneinander für lineare oder verzweigte, gesättigte oder ungesättigte Kohlenwasserstoffreste mit 1 bis 25 Kohlenstoffatomen stehen, mit der Maßgabe, dass die Summe der Kohlenstoffatome von R' + R" nicht über 26 hinausgeht, wobei R' und R" gegebenenfalls durch eine Hydroxygruppe (-OH); Sulfongruppe ($-SO_3$); Sulfatgruppe ($-SO_4$); Phosphatgruppe ($-PO_4H_2$); primäre Amingruppe ($-NH_2$); sekundäre Amingruppe ($-NHR_1$) oder tertiäre Amingruppe ($-NR_1R_2$) und/oder quaternäre Amingruppe ($-N^+R_1R_2R_3$) substituiert sind, wobei $R_1$, $R_2$ und $R_3$ unabhängig voneinander für einen linearen oder verzweigten, gesättigten oder ungesättigten Kohlenwasserstoffrest mit

1 bis 25 Kohlenstoffatomen stehen, mit der Maßgabe, dass die Summe der Kohlenstoffatome von $R_1$ + $R_2$ nicht über 26 hinausgeht und dass die Summe der Kohlenstoffatome von $R_1$ + $R_2$ +$R_3$ nicht über 27 hinausgeht;

ausgewählt ist;
- Maleinsäureanhydrid;
- Itaconsäure;
- Vinylalkohol der Formel $CH_2$=CHOH;
- Vinylacetat der Formel $CH_2$=CH-OCO$CH_3$ ausgewählt sind.

**4.** Zusammensetzung nach Anspruch 3, wobei die wasserlöslichen Einheiten außerdem durch Polymerisation von mindestens einem Monomer B als solchem oder in Form eines organischen oder anorganischen Salzes erhalten werden, wobei die Monomere B allein oder als Mischung unter:

- Vinylmonomeren der folgenden Formel (IIb):

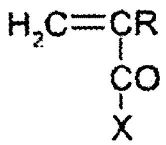

(IIb)

worin:
- R unter H, -$CH_3$, -$C_2H_5$ oder -$C_3H_7$ ausgewählt ist,
- X unter Alkyloxiden vom Typ -OR', wobei R' für einen linearen oder verzweigten, gesättigten oder ungesättigten Kohlenwasserstoffrest mit 1 bis 8 Kohlenstoffatomen steht, gegebenenfalls durch eine Sulfongruppe (-$SO_3$); Sulfatgruppe (-$SO_4$); Phosphatgruppe (-$PO_4H_2$) und/oder quaternäre Amingruppe (-$N^+R_1R_2R_3$) substituiert sind, wobei $R_1$, $R_2$ und $R_3$ unabhängig voneinander für einen linearen oder verzweigten, gesättigten oder ungesättigten Kohlenwasserstoffrest mit 1 bis 25 Kohlenstoffatomen stehen, mit der Maßgabe, dass die Summe der Kohlenstoffatome von $R_1$ + $R_2$ + $R_3$ nicht über 27 hinausgeht, ausgewählt ist;
- N-Vinyllactamen wie N-Vinylpyrrolidon, N-Vinylcaprolactam und N-Butyrolactam;
- Vinylethern der Formel $CH_2$=CHOR, worin R für einen linearen oder verzweigten, gesättigten oder ungesättigten Kohlenwasserstoffrest mit 1 bis 25 Kohlenstoffatomen steht,
- wasserlöslichen Styrolderivaten, insbesondere Styrolsulfonat;
- Dimethyldiallylammoniumchlorid;
- Vinylacetamid ausgewählt sind.

**5.** Zusammensetzung nach einem der Ansprüche 1 bis 2, wobei die wasserlöslichen Einheiten unter:

- wasserlöslichen Polyurethanen mit mindestens zwei reaktiven Stellen, insbesondere mit Carbonsäurefunktionen,
- Xanthan,
- Alginaten und Derivaten davon wie Propylenglykolalginat,
- Cellulosederivaten und insbesondere Carboxymethylcellulose, Hydroxypropylcellulose, Hydroxyethylcellulose und quaternisierter Hydroxyethylcellulose;
- Galactomannanen und Derivaten davon, wie Konjak-Gummi, Guar-Gummi, Hydroxypropylguar, mit Natriummethylcarboxylatgruppen modifiziertem Hydroxypropylguar und Hydroxypropyltrimethylammoniumguarchlorid;
- Polyethylenimin

ausgewählt sind.

**6.** Zusammensetzung nach einem der vorhergehenden Ansprüche, wobei die wasserlöslichen Einheiten eine Molmasse zwischen 50.0000 g/mol und 5.000.000 g/mol aufweisen, wenn sie die wasserlösliche Hauptkette eines Pfropfpolymers bilden, oder eine Molmasse zwischen 5000 g/mol und 100.000 g/mol aufweisen, wenn sie einen Block eines Mehrblockpolymers bilden.

**7.** Zusammensetzung nach einem der vorhergehenden Ansprüche, wobei die Einheiten mit LCST aus statistisch aufgebauten Copolymeren von Ethylenoxid und Propylenoxid der Formel $(EO)_m(PO)_n$, worin m für eine Zahl zwischen 1 und 20 inklusive steht und n für eine Zahl zwischen 20 und 50 inklusive steht, bestehen.

**8.** Zusammensetzung nach einem der vorhergehenden Ansprüche, wobei die Molmasse der Einheiten mit LCST zwischen 1500 und 5300 g/mol, insbesondere zwischen 2000 und 4000 g/mol, liegt.

**9.** Zusammensetzung nach einem der vorhergehenden Ansprüche, wobei die Einheiten mit LCST in Form von aminierten, insbesondere monoaminierten, diaminierten oder triaminierten, statistisch aufgebauten Copolymeren von Ethylenoxid und Propylenoxid vorliegen.

**10.** Zusammensetzung nach einem der vorhergehenden Ansprüche, wobei der Massenanteil der Einheiten mit LCST im fertigen Polymer zwischen 5 und 70 Gew.-%, inbesondere zwischen 20 und 65 Gew.-% und speziell zwischen 30 und 60 Gew.-%, bezogen auf das fertige Polymer, liegt.

**11.** Zusammensetzung nach einem der vorhergehenden Ansprüche, wobei die Erwärmungsentmischungstemperatur vom LCST-Typ der Einheiten mit LCST für eine Massenkonzentration der Einheiten mit LCST in Wasser von 1 Gew.-% zwischen 5°C und 40°C, vorzugsweise 10°C und 35°C, liegt.

**12.** Zusammensetzung nach einem der vorhergehenden Ansprüche, wobei das Polymer eine Wasserlöslichkeit bei 20°C von mindestens 10 g/l, vorzugsweise mindestens 20 g/l, aufweist.

**13.** Zusammensetzung nach einem der vorhergehenden Ansprüche, die in Form einer verdickten oder sogar gelierten wässrigen Zusammensetzung vorliegt, die unabhängig von der zwischen 400 und 800 nm liegenden Wellenlänge einen Wert der maximalen Lichtdurchlässigkeit durch eine Probe mit einer Dicke von 1 cm von mindestens 90% und vorzugsweise mindestens 85% aufweist.

**14.** Zusammensetzung nach einem der vorhergehenden Ansprüche, wobei die Polymere in einer Menge zwischen 0,05 und 15 Gew.-% und insbesondere 0,1 bis 10 Gew.-% vorliegen.

**15.** Zusammensetzung nach einem der vorhergehenden Ansprüche, die außerdem ein kosmetisch oder pharmazeutisch unbedenkliches Medium umfasst.

**16.** Zusammensetzung nach einem der vorhergehenden Ansprüche, die in Form einer kosmetischen Makeup- oder Pflegezusammensetzung, die auf die Haut, die Nägel, die Haare, die Wimpern, die Augenbrauen, die Schleimhäute und die Halbschleimhäute und jede andere Körper- oder Gesichtshautzone aufgebracht werden kann.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0583814 A
- EP 0629649 A
- WO 9831643 A
- WO 9524430 A
- WO 9700275 A
- EP 291334 A


### Littérature non-brevet citée dans la description


- **TAYLOR et al.** *Journal of Polymer Science, part A : Polymer Chemistry,* 1975, vol. 13, 2551
- **BAILEY et al.** *Journal of Applied Polymer Science,* 1959, vol. 1, 56
- **HESKINS et al.** *Journal of Macromolecular Science, Chemistry,* 1968, vol. A2, 1441
- **HOURDET et al.** Synthesis of thermoassociative copolymers. *Polymer,* vol. 38 (10), 2535-2547
- **HOURDET et al.** *Polymer,* 1997, vol. 38 (10), 2535-2547